# EUROPEAN PATENT SPECIFICATION

(11) **EP 3 586 743 B1**
(45) Date of publication and mention of the grant of the patent: **10.12.2025**
(21) Application number: 18180754.6
(22) Date of filing: 29.06.2018
(51) Int. Cl.: A61B 5/11, A61B 5/00

(54) **METHOD AND APPARATUS FOR DETERMINING INFORMATION REGARDING BODY POSITION**
VERFAHREN UND VORRICHTUNG ZUR BESTIMMUNG VON INFORMATIONEN ÜBER DIE KÖRPERPOSITION
PROCÉDÉ ET APPAREIL PERMETTANT DE DÉTERMINER DES INFORMATIONS CONCERNANT LA POSITION DU CORPS

(43) Date of publication of application: 01.01.2020
(73) Proprietor: Nokia Technologies Oy, 02610 Espoo (FI)
(72) Inventor: MATIC, Tomislav, 31000 Osijek (HR); HERCEG, Marijan, 31000 Osijek (HR); GRBIC, Ratko, 31000 Osijek (HR)
(74) Representative: Swindell & Pearson Limited

(56) References cited:
- EP-A1- 2 783 725
- US-A1- 2016 313 798
- US-A1- 2017 147 079

## Description

### TECHNOLOGICAL FIELD

An example embodiment relates generally to a method, apparatus and computer program product for determining information regarding the position of at least a portion of the body of a subject and, more particularly, to a method, apparatus and computer program product for determining information regarding the position of at least a portion of the body of the subject based upon a signal transmitted via capacitively-coupled intra body communication channels.

### BACKGROUND

Motion estimation and/or body posture detection is required for a number of applications including, for example, activity tracking applications and health care monitoring systems. For example, at least some fall detectors and step counters rely upon motion estimation and/or body posture detection in order to detect a fall or a step.

Various techniques have been developed in order to provide for motion estimation and body position detection. For example, image processing systems, ultrasonic systems, infrared systems, radio frequency identification (RFID) systems, radio frequency (RF) systems, electric field sensing systems, capacitive-sensing systems, accelerometer-based systems and camera-based systems have been developed in order to provide for motion estimation and/or body position detection. However, a number of these systems may be relatively complex and relatively expensive. In addition, some systems for providing motion estimation and/or body posture detection may require substantial input power and may be relatively inefficient in terms of energy consumption. Additionally, accelerometer-based systems may be sensitive to noise sources, such as breathing or coughing, which may adversely impact the resulting motion estimation and/or body posture detection. Further, camera-based systems may be sensitive to the color of clothing worn by the subject and/or the disposition of the camera relative to the subject such that the resulting motion estimation and/or body posture detection may vary depending upon clothing color and/or camera disposition.

Further, capacitive-sensing systems measure the capacitance between nodes upon the subject's body. Based upon the measured capacitance, the distance between the nodes is derived in order to provide for motion estimation and body posture detection. However, the capacitance measurements may sometimes lack sufficient resolution and may be inaccurate in some instances.

US 2017/0147079 A1 discloses a human body-based interaction method and interaction apparatus.

US 2016/0313798 A1 discloses a wearable device or system for measuring finger motion and recognizing hand gestures.

### BRIEF SUMMARY

The scope of protection sought for various embodiments of the invention is set out by the independent claims. The examples and features, if any, described in this specification that do not fall under the scope of the independent claims are to be interpreted as examples useful for understanding various embodiments of the invention. A method, apparatus, computer program product and system are provided in order to determine the position of a least a portion of the body of a subject, thereby providing for motion estimation and/or body posture detection. The method, apparatus, computer program product and system of an example embodiment determine information regarding the position of at least a portion of the body of the subject based upon a signal that propagated through one or more capacitively-coupled intra body communication channels. As such, the method, apparatus, computer program product and system of an example embodiment determine information regarding the position of at least a portion of the body in an energy efficient manner utilizing a relatively low power signal that propagates through the capacitively-coupled intra body communication channel(s). Moreover, the method, apparatus, computer program product and system of an example embodiment estimate motion and detect body posture in an accurate and reliable manner that is insensitive to a number of external factors, such as the clothing worn by the subject and noise including breathing or coughing of the subject.

In an example embodiment, an apparatus is provided that include means for receiving information regarding a plurality of signals that propagated between pairs of nodes through a plurality of capacitively-coupled intra body communication channels in a body. The apparatus also includes means for evaluating the information regarding the plurality of signals in order to determine information regarding a position of at least a portion of the body by evaluating envelopes of the plurality of signals that propagated through the plurality of capacitively-coupled intra body communication channels that vary in a manner attributable to changes in capacitance between the nodes.

In an example embodiment, the means for evaluating is configured to evaluate a strength of the plurality of signals in order to determine information regarding the position of at least the portion of the body. The strength of the plurality of the signals varies based upon changes in capacitance between the nodes when at least the portion of the body is in the different positions. In an example embodiment, the means for evaluating is configured to evaluate a phase delay of the plurality of signals in order to determine information regarding the position of at least the portion of the body. The phase delay of the plurality of signals varies based upon changes in capacitance between the first and second nodes when at least the portion of the body is in the different positions.

In an example embodiment, the means for evaluating is configured to evaluate the information regarding the plurality of signals that propagated through the capacitively-coupled intra body communication channels at different instances in time in order to determine information regarding movement of the body. In this example embodiment, the means for evaluating may be further configured to evaluate changes in the plurality of signals that propagated through the plurality of capacitively-coupled intra body communication channels at the different instances in time in order to determine information regarding movement of the body. In this example embodiment, the means for evaluating may be further configured to identify the movement of the body based upon a comparison of the information regarding the plurality of signals that propagated through the plurality of capacitively-coupled intra body communication channels at the different instances in time to a plurality of patterns associated with respective movements of the body.

In an example embodiment, the means for receiving is configured to receive information regarding a plurality of signals that propagated through a plurality of capacitively-coupled intra body communication channels through the body. In this example embodiment, the means for evaluating is configured to evaluate the information regarding the signals that propagated through the plurality of capacitively-coupled intra body communication channels in order to determine information regarding the position of at least the portion of the body. In this example embodiment, the means for evaluating may be further configured to evaluate differences in the signals that propagated through the plurality of capacitively-coupled intra body communication channels in order to determine information regarding the position of at least the portion of the body. In this example embodiment, the means for evaluating may be further configured to identify the position of at least the portion of the body based upon a comparison of the information regarding the plurality of signals that propagated through the plurality of capacitively-coupled intra body communication channels through the body to a plurality of patterns associated with respective positions of at least the portion of the body. In this example embodiment, the plurality of capacitively-coupled intra body communication channels through the body may include a plurality of capacitively-coupled intra body communication channels through the body that originate with different sources. In this example embodiment, the means for evaluating is further configured to distinguish the signals that originate with the different sources based on a characteristic of the signals that is different for the signals transmitted by the respective sources.

In another example embodiment, an apparatus is provided that includes at least one processor and at least one memory including computer program code with the at least one memory and the computer code configured to, with the at least one processor, receive information regarding a signal that propagated through a capacitively-coupled intra body communication channel in the body of a subject from a first node to a second node and evaluate the information regarding the signal in order to determine information regarding the position of at least a portion of the body. In this regard, the at least one memory and the computer program code are configured to, with the at least one processor, cause evaluation of the information to identify different positions of at least the portion of the body based upon the plurality of signals that propagated through the capacitively-coupled intra body communication channels that vary in a manner attributable to changes in capacitance between the first and second nodes.

In a further example embodiment, a method is provided that includes receiving information regarding a plurality of signals that propagated between pairs of nodes through a plurality of capacitively-coupled intra body communication channels through the body of a subject. The method also includes evaluating the information regarding the plurality of signals in order to determine information regarding a position of at least a portion of the body. In this regard, the method evaluates the information regarding the signal by identifying different positions of at least the portion of the body by evaluating envelopes of the plurality of signals that propagated through the capacitively-coupled intra body communication channels that vary in a manner attributable to changes in capacitance between the nodes. In an example embodiment, the method evaluates the information regarding the signal by evaluating the information regarding the plurality of signals that propagated through the plurality of capacitively-coupled intra body communication channels at different instances in time in order to determine information regarding movement of the body.

In yet another example embodiment, a computer-readable medium is provided for storing a computer program comprising computer program instructions configured, when working with at least one processor, to cause an apparatus at least to receive information regarding a plurality of signals that propagated between pairs of nodes through a plurality of capacitively-coupled intra body communication channels in the body. The computer program instructions are also configured, when working with the at least one processor, to cause the apparatus to evaluate the information regarding the plurality of signals in order to determine information regarding a position of at least a portion of the body. In this regard, the computer program instructions are configured, when working with at least one processor, to cause the apparatus to evaluate the information regarding the signal by identifying different positions of at least the portion of the body by evaluating envelopes of the plurality of signals that propagated through the plurality of capacitively-coupled intra body communication channels that vary in a manner attributable to changes in capacitance between the nodes.

In another example embodiment, a system is provided that includes means for transmitting a signal via a capacitively-coupled intra body communication channel through the body of a subject. The system also includes means for receiving a signal transmitted via the capacitively-coupled intra body communication channel. The system further includes means for evaluating information regarding the signal that was received in order to determine information regarding a position of at least a portion of the body. The means for evaluating is configured to identify different positions of at least the portion of the body based upon the signal that propagated through the capacitively-coupled intra body communication channel that varies in a manner attributable to changes in capacitance of the intra body communication channel.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 is a schematic representation of a system configured to identify different positions of at least the portion of the body of a subject in accordance with an example embodiment;
FIG. 2 is a cross-sectional view of a pair of electrodes configured to transmit or receive signals via the intra body communication channel in accordance with an example embodiment of the present disclosure;
FIG. 3 illustrates an example of the means for transmitting signals via an intra body communication channel and the means for receiving signals via the intra body communication channel in accordance with an example embodiment of the present disclosure;
FIG. 4 is a flow chart illustrating operations performed, such as by the system of FIG. 1, in order to determine information regarding a position of at least a portion of the body of a subject in accordance with an example embodiment;
FIGs. 5A and 5B depict two alternative configurations of the nodes for transmitting and receiving signals via the intra body communication channel in accordance with an example embodiment of the present disclosure;
FIG. 6 is a flow chart illustrating operations performed, such as by the apparatus of FIG. 1, in order to determine information regarding different positions of at least a portion of the body of a subject in accordance with an example embodiment;
FIG. 7 is a flow chart illustrating operations performed, such as by the apparatus of FIG. 1, in order to identify movement of the body of a subject in accordance with an example embodiment;
FIGs. 8A-8C depict the intra body communication channel through a portion of the body of the subject in seated, standing and lying positions, respectively;
FIG. 9 is a graphical representation of the envelope of the signal transmitted by node S₁ and received at node S₂ in the seated, standing and lying positions depicted in FIGs. 8A-8C;
FIG. 10 is a flow chart illustrating operations performed, such as by the apparatus of FIG. 1, in order to determine information regarding a position of at least a portion of the body of a subject in accordance with an example embodiment;
FIGs. 11A-11C depict the intra body communication channel through a portion of the body of the subject as the subject is walking; and
FIG. 12 is a graphical representation of the envelope of the signal transmitted by node S₁ and received at node S₂ as the subject is walking as depicted in FIGs. 11A-11C.

### DETAILED DESCRIPTION

Some embodiments of the present invention will now be described more fully hereinafter with reference to the accompanying drawings, in which some, but not all, embodiments of the invention are shown. Indeed, various embodiments of the invention may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present invention.

Additionally, as used herein, the term 'circuitry' may refer to one or more or all of the following: (a) hardware-only circuit implementations (such as implementations in analog circuitry and/or digital circuitry); (b) combinations of circuits and software, such as (as applicable): (i) a combination of analog and/or digital hardware circuit(s) with software/firmware and (ii) any portions of hardware processor(s) with software (including digital signal processor(s)), software, and memory(ies) that work together to cause an apparatus, such as a mobile phone or server, to perform various functions) and (c) hardware circuit(s) and/or processor(s), such as a microprocessor(s) or a portion of a microprocessor(s), that requires software (e.g., firmware) for operation, but the software may not be present when needed for operation. This definition of 'circuitry' applies to all uses of this term in this application, including in any claims. As a further example, as used in this application, the term 'circuitry' also covers an implementation of merely a hardware circuit or processor (or multiple processors) or portions of a hardware circuit or processor and its (or their) accompanying software and/or firmware. The term 'circuitry' also covers, for example and if applicable to the particular claim element, a baseband integrated circuit or processor integrated circuit for a mobile phone or a similar integrated circuit in a server, a cellular network device or other computing or network device.

As defined herein, a "computer-readable storage medium," which refers to a physical storage medium (e.g., volatile or non-volatile memory device), may be differentiated from a "computer-readable transmission medium," which refers to an electromagnetic signal.

A method, apparatus, computer program product and system are provided in accordance with an example embodiment in order to provide for motion estimation and/or body posture detection. The motion estimation and/or body posture detection may be employed by any of a wide variety of applications including, for example, activity tracking applications, healthcare monitoring systems including, for example, fall detectors, step counters or the like, interactive gaming and/or virtual reality applications. As described hereinafter, the method, apparatus, computer program product and system of an example embodiment evaluate a signal transmitted via one or more capacitively-coupled intra body communication channels and, as such, provide for motion estimation and/or body posture detection in an energy efficient manner as a result of the relatively low power requirements for the transmission and reception of signals via one or more capacitively-coupled intra body communication channels. As a result of the reliance on a signal transmitted by one or more capacitively-coupled intra body communication channels, the method, apparatus, computer program product and system of an example embodiment have increased insensitivity to noise, such as breathing or coughing by the subject, and to the color of clothing worn by the subject. However, since the method, apparatus, computer program product and system of an example embodiment do not require the capacitance of a capacitively-coupled intra body communication channel to be determined, the accuracy of the resulting motion estimation and/or body posture detection may also be enhanced.

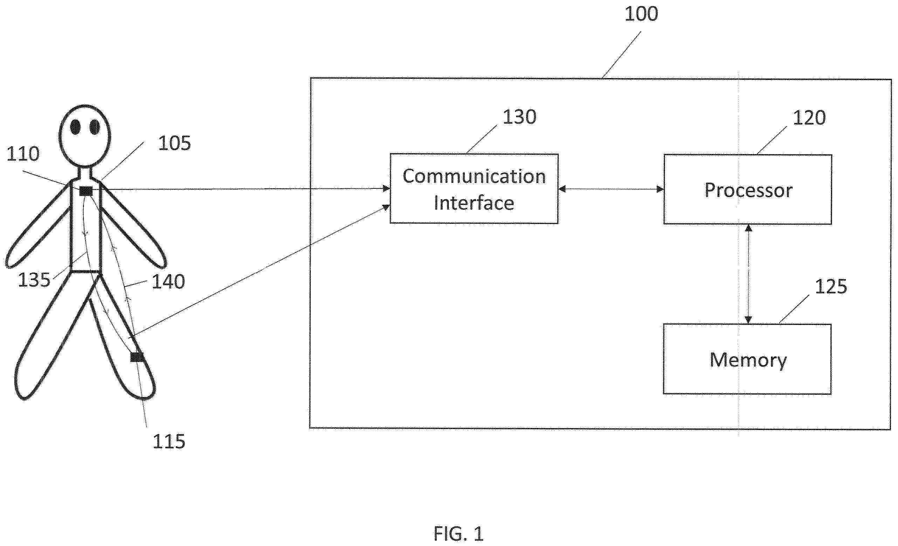

A system for determining information regarding the position of at least a portion of the body, such as for motion estimation and/or body posture detection, is depicted in accordance with an example embodiment with reference to FIG. 1. As shown, the system of this example embodiment includes an apparatus 100 that is configured as described below to evaluate the signal that propagates through one or more capacitively-coupled intra body communication channels and to determine information regarding the position of at least a portion of the body of a subject 105. As shown, the subject 105 may be a person. However, the subject 105 may, instead, be an animal whose movement is monitored, for example, for rehabilitation purposes.

The system of this example embodiment also includes means for transmitting a signal via a capacitively-coupled intra body communication channel through the body of the subject 105. The means for transmitting the signal may be embodied by a first node in the form of a transmitter, a transceiver or the like including one or more first electrodes. Further, this example embodiment includes means for receiving this signal transmitted via the capacitively-coupled intra body communication channel. Although the means for receiving the signal may be embodied in various manners, the means for receiving the signal in accordance with one example embodiment following propagation through the capacitively-coupled intra body communication channel is a second node in the form of a receiver, a transceiver or the like including one or more second electrodes. In some embodiments, the means for receiving the signal and/or the apparatus 100 in communication therewith associates a time of receipt with the signal that is received. In this regard, an indication of the time of receipt of the signal by the second node may be associated with the signal that is received (or a representation of the signal that is received), such as by storing the time of receipt of the signal along with or otherwise in association with the signal (or a representation of the signal) that are received. Thus, the received signal is effectively time stamped.

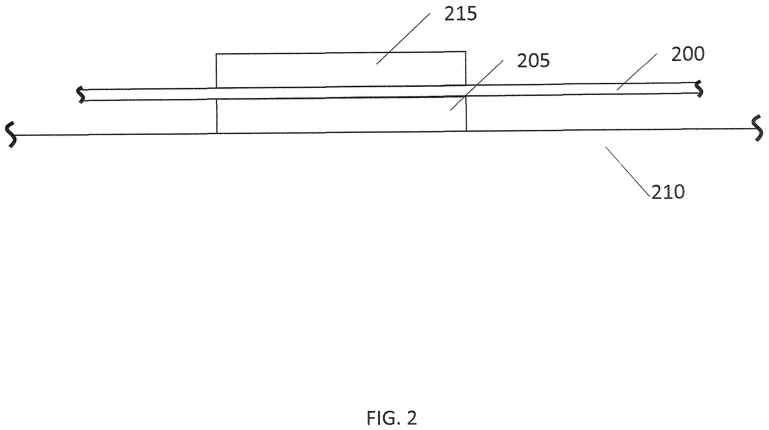

In an embodiment as shown in FIG. 1 and as described below for purposes of example, but not of limitation, in which the transmitter and the receiver of the first and second nodes, respectively, include first and second electrode pairs 110, 115, respectively, to transmit and receive signals via the capacitively-coupled intra body communication channel, the first and second electrode pairs may be attached to the subject 105, such as by taping or otherwise releasably adhering at least one electrode of each electrode pair to the skin of the subject. In an example embodiment, one electrode, e.g., the signal electrode, of an electrode pair may be taped to the skin of the subject, while the other electrode, e.g., the ground electrode, of the electrode pair is spaced from and floating relative to the skin of the subject. For example, the ground electrode may be stacked upon the signal electrode with a layer of insulation there between. In some embodiments, a couplant, such as a gel, is applied between the at least one electrode of each electrode pair 110, 115 that is attached to the subject 105 and the skin of the subject in order to improve the coupling of the signal there between. In other embodiments, the electrode pairs 110, 115 are not attached to the subject 105, but are included within and carried by a fabric, e.g., a smart fabric, which is worn by the subject so as to cause the electrodes to be positioned in the desired locations and in operable contact with the subject. Although the electrode pairs carried by a fabric are not attached to the subject, each electrode pair of this example embodiment may include one electrode, e.g., the signal electrode, carried by an inner surface of the fabric facing the subject so as to be in contact with the subject, while the other electrode, e.g., the ground electrode, is disposed within the fabric or carried by an outer surface of the fabric facing away from the subject such that the other electrode is not in contact with the subject but, instead, is floating in an instance in which the fabric is comprised of an insulative material. See, for example, Figure 2 in which the fabric 200 is an insulative material and serves to separate one electrode 205, e.g., a signal electrode, of the electrode pair that is in contact with the subject 210 from the other electrode 215, e.g., a ground electrode, of the electrode pair. Although the electrode pairs 110, 115 may be attached to different portions of the subject as described in several subsequent examples, the electrode pairs are generally spaced apart from one and other and are attached to different portions of the body, e.g., different body parts, that move relative to one another as the subject assumes different positions or postures or otherwise moves.

As shown in FIG. 1, an intra body communication channel 135 extends through the body of the subject between the first and second nodes, such as between the first and second electrode pairs 110, 115. As also shown in FIG. 1, an electric field 140 is also established through the air between the first and second electrode pairs 110, 115. As explained below, the strength of the electric field and correspondingly the strength of the signal transmitted between the first and second electrode pairs 110, 115 is dependent upon the capacitance experienced by the electric field between the first and second electrode pairs which, in turn, is dependent upon the position of the body or at least those portions of the body upon which the first and second electrode pairs are attached.

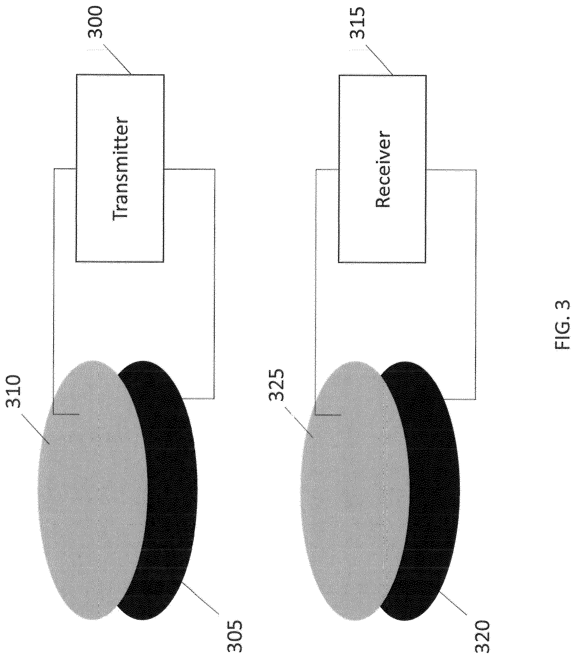

Referring now to FIG. 3, one example of the means for transmitting a signal via a capacitively-coupled intra body communication channel and the means for receiving a signal via the capacitively-coupled intra body communication channel is depicted. Although the first and second nodes may include any number of electrodes, the means for transmitting of the illustrated embodiment includes a transmitter 300 coupled between a first electrode pair. The first electrode pair includes a signal electrode 305 and a ground electrode 310. At least one of the electrodes, such as the signal electrode, is attached to subject as described above. The other electrode may be positioned in parallel to the electrode attached to the subject, such as by being placed upon the electrode attached to the subject with a layer of electrical insulation there between, so as to be floating. Similarly, the means for receiving of the illustrated embodiment includes a receiver 315 coupled between a second electrode pair. The second electrode pair also includes a signal electrode 320 and a ground electrode 325. At least one of the electrodes, such as the signal electrode, is attached to the subject as described above. The other electrode may be positioned in parallel to the electrode attached to the subject, such as by being placed upon the electrode attached to the subject with a layer of electrical insulation there between, so as to also be floating.

The means for transmitting the signal, such as the transmitter 300, and the means for receiving the signal, such as the receiver 315, may be battery powered and/or may employ energy harvesting to supply at least some of its power requirements. The means for transmitting the signal, such as the transmitter 300, and the means for receiving the signal, such as the receiver 315, are in communication with the apparatus 100. Although the means for transmitting the signal, such as the transmitter 300, and the means for receiving this signal, such as the receiver 315, may be in communication with the apparatus 100 via wired connection, the means for transmitting the signal, such as the transmitter 300, and the means for receiving the signal, such as the receiver 315, may be in wireless communication, such as via a Bluetooth^{®} Low Energy (BTLE) connection, with the apparatus 100, such as shown in FIG. 1. Thus, the apparatus 100 of this example embodiment is configured to receive information regarding the signal that has been received following propagation through the capacitively-coupled intra body communication channel.

The apparatus 100 may be embodied by or in communication with any of a variety of different types of computing devices including, for example, a mobile computing device, such as a smart phone or other mobile telephone, a tablet computer, a laptop computer, an activity tracker, a smart watch or other wearable computing device, a health monitoring device, a sleep monitoring device or the like. As shown in Figure 1, the apparatus 100 of an example embodiment includes, is associated with or is otherwise in communication with a processor 120, an associated memory 125 and a communication interface 130.

The processor 120 (and/or co-processors or any other circuitry assisting or otherwise associated with the processor) may be in communication with the memory device 125 via a bus for passing information among components of the apparatus 100. The memory device 125 may be non-transitory and may include, for example, one or more volatile and/or non-volatile memories. In other words, for example, the memory device 125 may be an electronic storage device (e.g., a computer readable storage medium) comprising gates configured to store data (e.g., bits) that may be retrievable by a machine (e.g., a computing device like the processor 120). The memory device 125 may be configured to store information, data, content, applications, instructions, or the like for enabling the apparatus 100 to carry out various functions in accordance with an example embodiment of the present disclosure. For example, the memory device 125 could be configured to buffer input data for processing by the processor 120. Additionally or alternatively, the memory device 125 could be configured to store instructions for execution by the processor 120.

The apparatus 100 may, in some embodiments, be embodied in various computing devices as described above. However, in some embodiments, the apparatus 100 may be embodied as a chip or chip set. In other words, the apparatus 100 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The apparatus 100 may therefore, in some cases, be configured to implement an embodiment of the present invention on a single chip or as a single "system on a chip." As such, in some cases, a chip or chipset may constitute means for performing one or more operations for providing the functionalities described herein.

The processor 120 may be embodied in a number of different ways. For example, the processor 120 may be embodied as one or more of various hardware processing means such as a coprocessor, a microprocessor, a controller, a digital signal processor (DSP), a processing element with or without an accompanying DSP, or various other circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor 120 may include one or more processing cores configured to perform independently. A multi-core processor may enable multiprocessing within a single physical package. Additionally or alternatively, the processor 120 may include one or more processors configured in tandem via the bus to enable independent execution of instructions, pipelining and/or multithreading.

In an example embodiment, the processor 120 may be configured to execute instructions stored in the memory device 125 or otherwise accessible to the processor. Alternatively or additionally, the processor 120 may be configured to execute hard coded functionality. As such, whether configured by hardware or software methods, or by a combination thereof, the processor 120 may represent an entity (e.g., physically embodied in circuitry) capable of performing operations according to an embodiment of the present disclosure while configured accordingly. Thus, for example, when the processor 120 is embodied as an ASIC, FPGA or the like, the processor may be specifically configured hardware for conducting the operations described herein. Alternatively, as another example, when the processor 120 is embodied as an executor of instructions, the instructions may specifically configure the processor to perform the algorithms and/or operations described herein when the instructions are executed. However, in some cases, the processor 120 may be a processor of a specific device (e.g., an activity tracker) configured to employ an embodiment of the present invention by further configuration of the processor by instructions for performing the algorithms and/or operations described herein. The processor 120 may include, among other things, a clock, an arithmetic logic unit (ALU) and logic gates configured to support operation of the processor.

The communication interface 130 may be any means such as a device or circuitry embodied in either hardware or a combination of hardware and software that is configured to receive and/or transmit data, such as by receiving information regarding the signal received by the second electrode 115. In this regard, the communication interface 130 may include, for example, an antenna (or multiple antennas) and supporting hardware and/or software for enabling communications with a wireless communication network. Additionally or alternatively, the communication interface 130 may include the circuitry for interacting with the antenna(s) to cause transmission of a signal via the antenna(s) or to handle receipt of a signal received via the antenna(s). In some environments, the communication interface 130 may alternatively or also support wired communication. As such, for example, the communication interface 130 may include a communication modem and/or other hardware/software for supporting communication via cable, digital subscriber line (DSL), universal serial bus (USB) or other mechanisms.

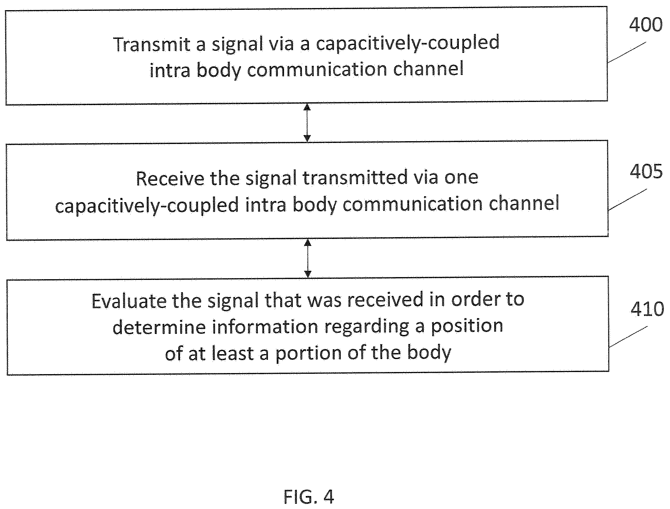

Referring now to FIG. 4, the operations performed, such as by the system of FIG. 1, are illustrated in accordance with an example embodiment. As shown in block 400 of FIG. 4, the system includes means, such as a first node including a transmitter 300, a transceiver or the like including one or more first electrodes, such as a first electrode pair 110, for transmitting a signal, such as a sinusoidal signal, via a capacitively-coupled intra body communication channel though the body of a subject. As described above in conjunction with an embodiment in which the first node includes a first electrode pair 110, the signal electrode 305 is attached to the subject and the ground electrode 310 is generally co-located with the signal electrode, but is floating relative to the subject, such as by being placed upon the signal electrode with an insulating material there between. A transmitter 300 or transceiver is connected between the first electrode pair 110 as shown in FIG. 3 such that the signal generated by the transmitter or transceiver is provided to the signal electrode and, in turn, to the subject. As the signal is transmitted via an intra body communication channel, the signal can be transmitted with a relatively low power such that the energy efficiency of the system is enhanced. In addition, the signal may be transmitted at a relatively low frequency, such as a frequency within a range of 1 MHz to 100 MHz. The transmission of the signal may be triggered in various manners, such as in accordance with a predefined schedule or at regular intervals. However, in some embodiments, the apparatus 100, such as the processor 120, is configured to initially direct the transmission of the signal, such as by the transmitter 300, via the capacitively-coupled intra body communication channel.

As shown in block 405 of FIG. 4, the system of this example embodiment also includes means, such as a second node including a receiver 315, a transceiver or the like including one or more second electrodes, such as a second electrode pair 115, for receiving the signal transmitted via the capacitively-coupled intra body communication channel. As described above in conjunction with an embodiment in which the second node includes a second electrode pair 115, the signal electrode 320 is attached to the subject and the ground electrode 325 is generally co-located with the signal electrode, but is floating relative to the subject, such as by being placed upon the signal electrode with an insulating material there between. As shown in FIG. 1, the first and second nodes including, for example, the first and second electrode pairs 110, 115, are connected to different parts of the subject so as to be spaced apart from one another. In an example embodiment, the first and second nodes including, for example, the first and second electrode pairs 110, 115 are connected to parts of the subject that will move and change position relative to one another in an instance in which the position or posture of the subject changes in a manner that would be desirable to detect. A receiver 315 or transceiver is connected between the second electrode pair 115 as shown in FIG. 3 such that the signal transmitted via the capacitively-coupled intra body communication channel is received by the signal electrode 320 and, in turn, by the receiver or transceiver. The amplitude of the signal received by the receiver 315, such as the maximum and minimum amplitudes of a sinusoidal signal, defines the envelope of the received signal. Although a single pair of first and second nodes is depicted in the example of FIG. 1, the system of other example embodiments may include additional pairs of first and second nodes attached to or otherwise carried by different body parts of the subject and associated with either the same transmitter 300 and receiver 315 or different transmitters and receivers in order to define a plurality of capacitively-coupled intra body communication channels though the body of the subject, thereby potentially increasing the accuracy and/or resolution with which body posture and/or motion estimation may be determined. Additionally, although described herein in conjunction with transmitter(s) that transmit a signal via respective intra body communication channels to receiver(s), transceivers may be utilized to both transmit and receive signals, such that a transceiver may both transmit a signal via an intra body communication channel and receive a different signal via, either the same or different, intra body communication channel.

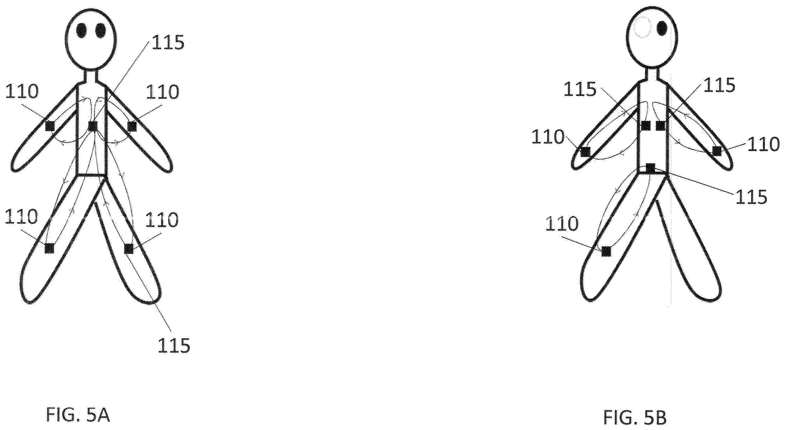

In an instance in which multiple intra body communication channels are defined, any one of several different architectures may be deployed. For example, the system may include a plurality of means for transmitting signals via different capacitively-coupled intra body communication channels through the body. In this example embodiment, a plurality of first nodes, such as a plurality of first electrode pairs 110, may be attached to different parts of the body and driven by one or more transmitters 300 to transmit signals via different intra body communication channels. In an example embodiment, a star topology may be utilized as shown in FIG. 5A in which the signals transmitted by the plurality of first nodes, such as the plurality of first electrode pairs 110, are received by the receiver 315 associated with a single second node, such as a single second electrode pair 115, thereby defining a plurality of different intra body communication channels, one of which extends between each first node and the second node. In an alternative embodiment, the system also includes a plurality of means for receiving signals following propagation through different capacitively-coupled intra body communication channels. In an alternative embodiment depicted in FIG. 5B, the plurality of means for receiving the signals may include a plurality of second nodes, such as a plurality of second electrode pairs 115, attached to different parts of the body. In this example embodiment, one or more receivers 315 associated with the plurality of second electrode pairs 115 receive signals transmitted by one or more first nodes, such as one or more first electrode pairs 110 via respective intra body communication channels. Regardless of the number of first nodes, a plurality of intra body communication channels are defined between the first node(s) and the plurality of second nodes.

In an embodiment of the system in which the plurality of capacitively-coupled intra body communication channels through the body originate with different sources, such as one or more transmitters 300 associated with different first electrode pairs 110, each of the plurality of means, such as the transmitter(s) 300, for transmitting a signal is configured to transmit a signal with a predefined characteristic that is distinct from the predefined characteristics of the signals transmitted by other one of the transmitters. Various characteristics may be utilized in order to uniquely identify the signal that originates with a respective source, such as a respective transmitter 300. For example, the signal may include a unique identifier of the source. Alternatively, the signal may be transmitted utilizing a frequency division multiplexing technique, a time division multiplexing technique and/or a code division multiplexing technique in order to provide unique characteristics for the signal transmitted by the respective source. Based upon the predefined characteristics, the respective intra body communication channel through which the signal is propagated may be identified.

Referring again to the embodiment of FIG. 1, a system of an example embodiment also includes means for evaluating the signal that was received, such as the envelope of the signal that was received, in order to determine information regarding the position of at least a portion of the body. See block 410 of FIG. 4. The means for evaluating the signal may be embodied in a variety of different manners, but in one embodiment, is embodied by the apparatus 100, such as the processor 120, as shown in FIG. 1 and as described above. The apparatus 100, such as the processor 120, is configured to evaluate different characteristics of the signal that is propagated via a respective intra body communication channel. For example, the apparatus 100, such as the processor 120, an example embodiment is configured to evaluate the strength of the signal in order to determine information regarding the position of at least the portion of the body. Additionally or alternatively, the apparatus 100, such as the processor 120, of another example embodiment is configured to evaluate the phase delay of the signal that is received following propagation via the intra body communication channel to determine information regarding the position of at least the portion of the body.

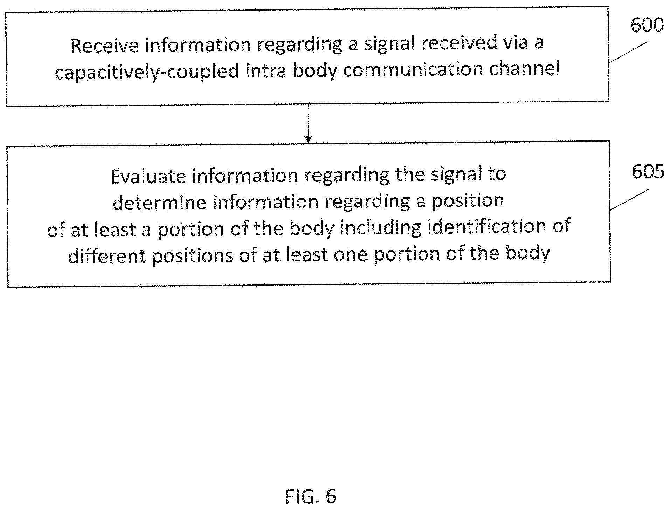

In this example embodiment, the apparatus 100 includes means, such as the communication interface 130, the processor 120 or the like, for receiving information regarding the signal that propagated through a capacitively-coupled intra body communication channel through the body. See block 600 of FIG. 6. As shown in the embodiment of FIG. 1, for example, the means, such as the receiver 315, for receiving the signal may provide information regarding the received signal to the apparatus 100, such as via the communication interface 130. The apparatus 100 of this example embodiment also include means, such as the processor 120 or the like, for evaluating the information regarding the signal, such as the envelope of the signal, in order to determine information regarding the position of at least a portion of the body. See block 605 of FIG. 6. In this regard, the means, such as the processor 120, for evaluating the information is configured to identify different positions of at least the portion of the body based upon differences in the signal that propagated through the capacitively-coupled intra body communication channel in a manner attributable to the changes in capacitance of the intra body communication channel. Thus, the signal that propagates through the intra body communication channel may be subjected to different amounts of capacitance depending upon the position of the body with the different capacitances causing the signal, such as the strength and/or phase delay of the signal, that is received to, in turn, be different. By way of example relative to FIG. 1 and as described in more detail below, first and second electrode pairs 110, 115 attached to the chest and a knee of the subject 105 establish an intra body communication channel there between. The capacitance experienced by a signal propagating through the intra body communication channel varies depending upon whether the subject 105 is standing as shown in FIG. 1 or is seated and, as a result, the signal received following propagation through the intra body communication channel correspondingly varies depending upon the capacitance and, in turn, upon the position of the body of the subject.

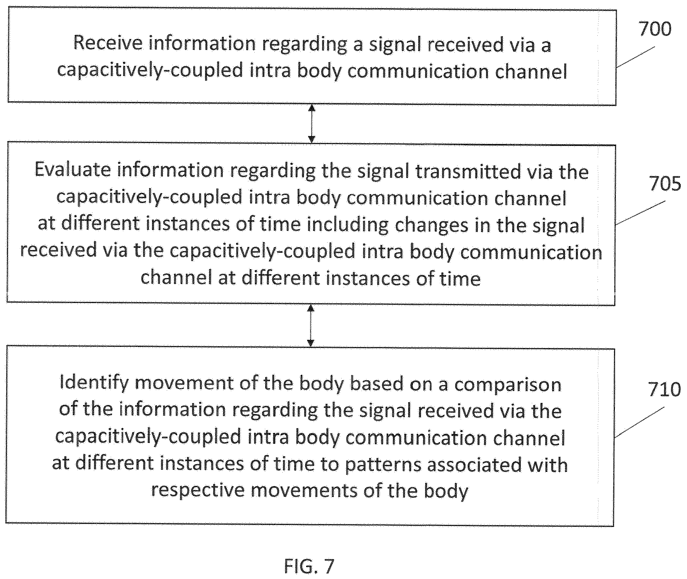

While the position of at least a portion of the body and, as a result, the body posture may be determined based upon the evaluation of signal transmitted via an intra body communication channel at a single instant in time, movement of the body, such as for motion estimation may be determined by repeatedly transmitting and receiving signals via the one or more intra body communication channels and evaluating changes in the signals propagating via the one or more intra body communication channels at the different instances in time. In this example embodiment and as depicted in the flowchart of FIG. 7, the apparatus 100 of an example embodiment includes means, such as the communication interface 130, the processor 120 or the like, for receiving information regarding signal(s) transmitted via one or more capacitively-coupled intra body communication channels through the body at different instances of time. See block 700 of FIG. 7. The apparatus 100 of this example embodiment also includes means, such as the processor 120 or the like, for evaluating information regarding the signal transmitted via the one or more capacitively-coupled intra body communication channels at the different instances of time. In this regard, the apparatus 100, such as the processor 120, is configured to evaluate the information regarding the signal transmitted via the one or more intra body communication channels at the different instances of time so as to evaluate changes in the signal transmitted via the one or more intra body communication channels at the different instances of time. See block 705 of FIG. 7. As shown in block 710, the apparatus 100 of this example embodiment also includes means, such as the processor 120 or the like, for identifying movement of the body based on the comparison of the information regarding signal transmitted via the one or more intra body communication channels at the different instances of time to patterns associated with respective movements of the body.

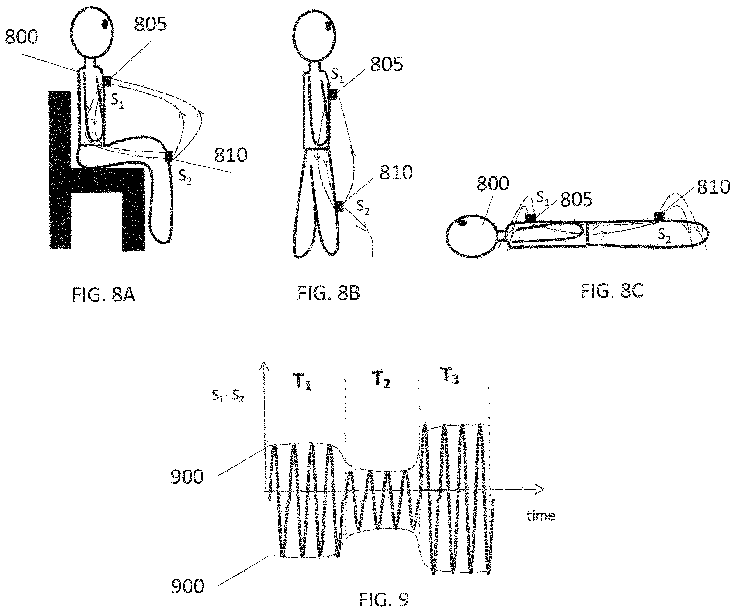

By way of example and as shown in FIG. 8A, a subject 800 has a first electrode pair 805 attached to their chest and a second electrode pair 810 attached to one of their knees. The subject of FIG. 8A is seated with their feet off the floor. A signal is transmitted by the transmitter 300 via the signal electrode 305 of the first electrode pair 805 through a capacitively-coupled intra body communication channel to a receiver 315 associated with the signal electrode 320 of the second electrode pair 810. To complete the circuit loop, an electric field is capacitively coupled from the ground electrode 325 of the second electrode pair 810 to the ground electrode 310 of the first electrode pair 805 via the air with a portion of the electric field, if any, distributed to ground as a result of the separation or spacing between the second electrode pair 810 and the ground.

As a point of contrast, FIG. 8B depicts the same subject 800 with the same first and second electrode pairs 805, 810, although the subject is now in a standing position. In this position, the signal transmitted by the transmitter 300 via the signal electrode 305 of the first electrode pair 805 again propagates via the intra body communication channel to the receiver 315 via the signal electrode 320 of the second electrode pair 810. An electric field is coupled between the ground electrode 325 of the second electrode pair 810 and the ground electrode 310 of the first electrode pair 805 through the air as described above, while portion of the electric field from the ground electrode 325 of the second electrode pair 810 is coupled to ground as a result of the closer proximity of the second electrode pair 810 to the ground when the subject is standing as opposed to when the subject is seated with their feet off the floor. In this regard, the distance through the air between the first and second electrode pairs 805, 810 increases when the subject 800 is in the standing position of FIG. 8B as opposed to the seated position in FIG. 8A such that the capacitive coupling between the first and second electrode pairs and, more particularly, between the ground electrodes 310, 325 of the first and second electrode pairs through the air is reduced when standing as opposed to being seated which results in increased impedance and an decrease in the strength of the electric field between the second electrode pair 810 and the first electrode pair 805. Thus, the signal that propagates via the intra body communication channel varies, such as by being reduced, as a result of the transition from the seated position to the standing position and the corresponding change, that is, decrease, in both the capacitance between the first and second electrode pairs and the strength of the electric field between the first and second electrode pairs.

As yet another example, FIG. 8C depicts the subject 800 lying on the ground. As before, the transmitter 300 transmits signals via the signal electrode 305 of the first electrode pair 110 that propagate via the intra body communication channel. As a result of the proximity of the first and second electrode pairs 805, 810 to ground, the capacitive coupling between the first and second electrode pairs and the ground is increased relative to the seated and standing positions of FIGs. 8A and 8B, respectively, such that a greater percentage of the electric field will be coupled between the electrode pairs and the ground plane, as opposed to coupling only through the air to the first electrode pair as in FIGs. 8A and 8B. Thus, the signal that propagates via the intra body communication channel varies, such as by being increased, as a result of the transition from the standing position to the lying position and the corresponding change, that is, increase, in both the capacitance between the first and second electrode pairs via the ground and the strength of the electric field between the first and second electrode pairs. The strength of the signal received by the first electrode pair 805 is therefore higher in the lying position than in the seated and standing positions.

In order to identify different positions and/or different movements of the body, information regarding the signals transmitted via the intra body communication channel, such as changes in the signal transmitted via the intra body communication channel, may be evaluated by the apparatus 100, such as the processor 120. As shown in FIG. 9, the different levels of the envelope 900 of the signal received by the receiver 315 via the signal electrode 320 of the second electrode pair 810, that were transmitted from the transmitter 300 via the signal electrode 305 of the first electrode pair 805, may be determined for different positions and/or different movements of the body. As shown in the graph of FIG. 9 in which T₁ represents the time period during which the subject is in a seated position as shown in FIG. 8A, T₂ represents the time period in which the subject is standing as shown in FIG. 8B and T₃ represents the time period in which the subject is lying on the ground as shown in FIG. 8C, the envelope 900 of the signal received by the signal electrode 320 of the second electrode pair 810 varies depending upon the position of the subject. In this regard, presuming that the capacitance of the intra body communication channel remains relatively constant in the different positions of FIGs. 8A-8C, the strength of the signal received by the receiver 315 associated with the second electrode pair 810 indicates that the signal has a greater strength when received by the second electrode pair during T₁ than during T₂ and that the signal has a lesser strength when received by the second electrode pair during T₁ and T₂ than during T₃. Thus, the information regarding the signal transmitted via the intra body communication channel, such as information regarding the envelope level of the signal received by the second electrode pair 810, can be evaluated by the apparatus 100, such as the processor 120, to identify the position of the subject 800, such as a seated position during the first time period T₁, a standing position in the second time period T₂ and a lying position in the time period T₃.

Further, the information regarding signals that has propagated via an intra body communication channel at different instances of time, such as changes in the signals transmitted via the intra body communication channel at different instances of time, can be evaluated by the apparatus 100, such as the processor 120, to permit movement of the body to be identified. For example, the apparatus 100, such as the processor 120, may determine that the subject 800 has stood as indicated by the change in the signal transmitted via the intra body communication channel between the first time period T₁ and the second time period T₂ or that the subject has laid down on the ground as indicated by changes in the signals transmitted via the intra body communication channel between the second time period T₂ and the third time period T₃.

In order to identify different positions of at least a portion of the body, such as for purposes of body posture detection or motion estimation, the apparatus 100, such as the processor 120, of an example embodiment is configured to compare the information regarding signal that has propagated via the intra body communication channel to patterns indicative of different positions of the body and/or different movements of the body. In this regard, the apparatus 100, such as the processor 120, of an example embodiment may be trained, such as by machine learning, in order to identify and recognize different patterns of the signal that propagates via an intra body communication channel that are indicative of different positions and/or different movements of the body. For example, electrode pairs may be attached to the subject in a predefined pattern, such as shown in FIGs. 1 and 8A-8C in which electrode pairs are attached to the chest and a knee of the subject, and the subject may then assume a variety of different positions. While the subject is in each position, a signal may be transmitted via the intra body communication channel(s) and information regarding the signal received following propagation via the intra body communication channel(s) may be collected and analyzed by the apparatus 100, such as the processor 120, so as to identify a predefined pattern that is indicative of the subject being in the respective position. Information regarding the different predefined patterns may be stored, for example, by the memory device 125. Different types of movement may also be identified by the apparatus 100, such as the processor 120, based upon a change from the pattern indicative of the subject being in a first position to a pattern indicative of the subject being in a different position. By storing these patterns, the apparatus 100, such as the processor 120, may subsequently compare the information regarding the signal that has propagated via a respective intra body communication channel to the predefined patterns in order to identify the predefined pattern that most closely matches the information regarding the signal that has propagated via the intra body communication channel. The apparatus 100, such as the processor 120, is then configured to identify the subject as being in the position associated with the matching pattern.

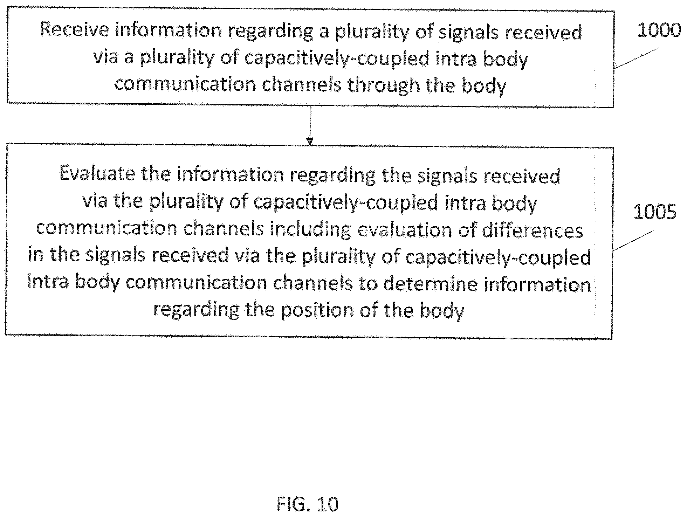

As noted above in conjunction with FIGs. 5A and 5B , the system of an example embodiment may include a plurality of means for transmitting signals via respective intra body communication channels and/or a plurality of means for receiving signals following propagation via respective intra body communication channels, such as in instances in which three or more electrode pairs are attached to different portions of the body of the subject. In this example embodiment, the apparatus 100 includes means, such as the communication interface 130, the processor 120 or the like, for receiving information regarding a plurality of signals transmitted via a plurality of capacitively-coupled intra body communication channels through the body. See block 1000 of FIG. 10. The apparatus 100 of this example embodiment includes means, such as the processor 120 or the like, for evaluating the signal propagating through a respective intra body communication channel and to distinguish the signals that originate with the different sources based upon the predefined characteristic of the signals that is unique to the source of the signals. Thus, the apparatus 100, such as the processor 120, is configured to identify the respective intra body communication channel through which the signal propagated based upon a predetermined relationship between the source of the signal (as identified by the unique signal characteristics) and the corresponding intra body communication channel through which the signal propagated.

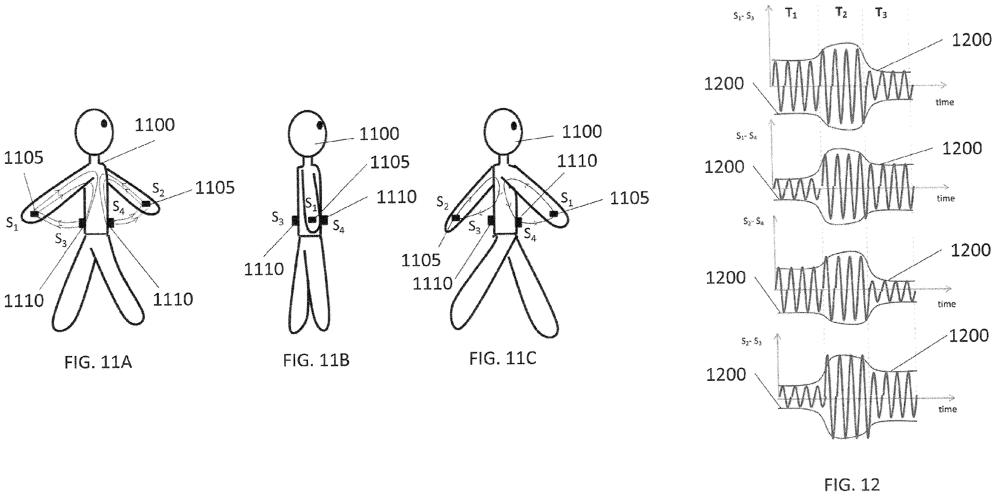

The apparatus 100 of this example embodiment also includes means, such as the processor 120 or the like, for evaluating the information regarding the signals transmitted via the plurality of capacitively-coupled intra body communication channels, including an evaluation of differences in the signals transmitted via the plurality of capacitively-coupled intra body communication channels, to determine information regarding the position of the body. See block 1005 of FIG. 10. By way of example, FIGs. 11A-11C illustrate a subject 1100 to which four electrode pairs are attached, with first electrode pairs 1105 attached to the left and right wrists and second electrode pairs 1110 attached to the abdomen and to the back of the subject. As shown in FIG. 11A, while the subject is walking with their arms swinging such that their left wrist is in front of their body and their right wrist is behind their body, a signal transmitted by the first electrode pair 1105 attached to the left wrist propagates via a first intra body communication channel through the left arm of the subject and then through the upper body of the subject to the second electrode pair 1110 attached to the abdomen of the subject. The first electrode pair 1105 attached to the left wrist is capacitively coupled through the air with the second electrode pair 1110 attached to the abdomen. Similarly, the signal transmitted by the first electrode pair 1105 attached to the right wrist propagates through a second intra body communication channel through the right arm of the subject and then through the upper body of the subject to the second electrode pair 1110 attached to the back of the subject. The first electrode pair 1105 attached to the right wrist is capacitively coupled through the air to the second electrode pair 1110 attached to the back of the subject.

Between strides, the arms of the subject 900 are at their sides as shown in FIG. 11B with the intra body communication channel from the first electrode pair 1105 attached to the left wrist extending through the left arm of the subject and then through the upper body of the subject to the second electrode pair 1110 that is the closest. Similarly, the signal transmitted by the first electrode pair 1105 attached to the right wrist propagates through an intra body communication channel that extends through the right arm of a subject and then to the second electrode pair 1110 that is the closest. The electric field from the second electrode pairs 1110 is coupled through the air to the respective first electrode pairs 1105, albeit with a much smaller air interface and much greater capacitive coupling than that shown in FIG. 11A. As shown in FIG. 11C, the next stride of the subject results in the left arm of the subject being behind their body and the right arm of the subject being in front of their body. Thus, the signal transmitted by the first electrode pair 1105 attached to the left wrist propagates through an intra body communication channel that extends through the left arm of the subject and through the upper body of the subject to the second electrode pair 1110 attached to the back of the subject, which is now closer to the first electrode attached to the left wrist. The electric field from the second electrode pair 1110 attached to the back of the subject is coupled through the air to the first electrode pair 1105 attached to the left wrist. Additionally, the signal transmitted by the first electrode pair 1105 attached to the right wrist propagates through an intra body communication channel that extends through the right arm of the subject and through the upper body of the subject to the second electrode pair 1110 attached to the abdomen of the subject, which is now closer to the first electrode pair attached to the right wrist. The electric field from the second electrode pair 1110 attached to the abdomen is coupled to the first electrode pair 1105 attached to the right wrist through the air.

As shown in FIG. 12, the envelopes 1200 of the signals received by different second electrode pairs 1110 during a first instance of time T₁ as shown in FIG. 11A, during a second instance of time T₂ as shown in FIG. 11B and during a third instance of time T₃ as shown in FIG. 11C are depicted. The envelope 1200 of the signal received by the second electrode pair 1110 on the back of the subject 1100, transmitted from the first electrode pair 1105 on the right wrist is denoted as S₁-S₃, the envelope of the signal received by the second electrode pair 1110 on the abdomen of the subject 1100, transmitted from the first electrode pair 1105 on the right wrist is denoted as S₁-S₄, the envelope of the signal received by the second electrode pair 1110 on the abdomen of the subject 1100, transmitted from the first electrode pair 1105 on the left wrist is denoted as S₂-S₄, and the envelope of the signal received by the second electrode pair 1110 on the back of the subject 1100, transmitted from the first electrode pair 1105 on the left wrist is denoted as S₂-S₃. The envelopes 1200 of the signals received by the various electrode pairs when the subject is in different positions is depicted in FIG. 12. Notably, for transmission of the signal from the electrode pair 1105 attached to the left wrist the sequence of envelopes (S₂ - S₄) of the signal received by the second electrode pair 1110 on the abdomen of the subject (intermediate envelope during T₁, larger envelope during T₂ and smaller envelope during T₃) is generally the opposite from the sequence of envelopes (S₂ - S₃) of the signal received by the second electrode pair 1110 on the back of the subject (smaller envelope during T₁, larger envelope during T₂ and intermediate envelope during T₃) from the same signal transmitted by the first electrode pair 1105 on the left wrist. Similarly, for transmission of the signal from the first electrode pair 1105 attached to the right wrist, the sequence of envelopes (S₁ - S₃) of the signal received by the second electrode pair 1110 on the back of the subject (intermediate envelope during T₁, larger envelope during T₂ and smaller envelope during T₃) is generally the opposite from the sequence of envelopes (S₁-S₄) of the signal received by the second electrode pair 1110 on the abdomen of the subject (smaller envelope during T₁, larger envelope during T₂ and intermediate envelope during T₃) from the same signal transmitted by the first electrode pair 1105 on the right wrist. Such symmetry in the sequences of the envelopes is indicative of walking in which the arms swing back and forth in a relatively symmetric manner.

Based upon a comparison of the signal patterns of FIG. 12 with patterns with which the system has been trained and which may be stored, for example, in memory 125, the apparatus 100, such as the processor 120, is configured to identify the pattern that most closely matches the envelopes 1200 of the signals shown in FIG. 12. The movement of the subject associated with the pattern that most closely matches the envelopes 1200 of the signals received by the various electrodes as shown in FIG. 12 is then identified. For example, the apparatus 100, such as the processor 120, may identify the person to be walking based upon the evaluation of the information regarding the signals propagating via the plurality of intra body communication channels and received by the various electrode pairs as shown in FIG. 12.

As described above, a method, apparatus 100, computer program product and system are provided in order to determine the position of a least a portion of the body of a subject, such as for motion estimation and/or body posture detection, based upon a signal that propagated through one or more capacitively-coupled intra body communication channels. As such, the method, apparatus, computer program product and system of an example embodiment are energy efficient by determining information regarding the position of at least a portion of the body utilizing relatively low power signals that propagate through the capacitively-coupled intra body communication channel(s). Moreover, the method, apparatus, computer program product and system of an example embodiment estimate motion and detect body posture in an accurate and reliable manner that is insensitive to a number of external factors, such as the clothing worn by the subject and noise created by the subject, but that is sensitive to even small changes in position.

As described above, FIGs. 4, 6, 7 and 10 are flowcharts of an apparatus 100, method, and computer program product according to certain example embodiments. It will be understood that each block of the flowcharts, and combinations of blocks in the flowcharts, may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by a memory device 125 of an apparatus employing an embodiment of the present invention and executed by processing circuitry or a processor 120 of the apparatus. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

A computer program product is therefore defined in those instances in which the computer program instructions, such as computer-readable program code portions, are stored by at least one non-transitory computer-readable storage medium with the computer program instructions, such as the computer-readable program code portions, being configured, upon execution, to perform the functions described above, such as in conjunction with the flowcharts of Figures 4, 6, 7 and 10. In other embodiments, the computer program instructions, such as the computer-readable program code portions, need not be stored or otherwise embodied by a non-transitory computer-readable storage medium, but may, instead, be embodied by a transitory medium with the computer program instructions, such as the computer-readable program code portions, still being configured, upon execution, to perform the functions described above.

Accordingly, blocks of the flowcharts support combinations of means for performing the specified functions and combinations of operations for performing the specified functions for performing the specified functions. It will also be understood that one or more blocks of the flowcharts, and combinations of blocks in the flowcharts, may be implemented by special purpose hardware-based computer systems which perform the specified functions, or combinations of special purpose hardware and computer instructions.

In some embodiments, certain ones of the operations above may be modified or further amplified. Furthermore, in some embodiments, additional optional operations may be included. Modifications, additions, or amplifications to the operations above may be performed in any order and in any combination.

## Claims

1. An apparatus (100) comprising:
means (120; 130; 315) for receiving information regarding a plurality of signals that propagated between pairs of nodes (110, 115; 305, 320; 805, 810; 1105, 1100) through a plurality of capacitively-coupled intra body communication channels in a body; and
means for evaluating the information regarding the plurality of signals in order to determine information regarding a position of at least a portion of the body, wherein the means for evaluating is configured to identify different positions of at least the portion of the body by evaluating envelopes (900; 1200) of the plurality of signals that propagated through the plurality of capacitively-coupled intra body communication channels that vary in a manner attributable to changes in capacitance between the nodes.

2. The apparatus according to claim 1, wherein the means for evaluating is configured to evaluate a strength of the plurality of signals in order to determine information regarding the position of at least the portion of the body, wherein the strength of the plurality of signals varies based upon changes in capacitance between the nodes when at least the portion of the body is in the different positions.

3. The apparatus according to any of claim 1 or 2, wherein the means for evaluating is configured to evaluate a phase delay of the plurality of signals in order to determine information regarding the position of at least the portion of the body, wherein the phase delay of the plurality of signals varies based upon changes in capacitance between the nodes when at least the portion of the body is in the different positions.

4. The apparatus according to any of claims 1 to 3, wherein the means for evaluating is configured to evaluate the information regarding the plurality of signals that propagated through the capacitively-coupled intra body communication channels at different instances in time in order to determine information regarding movement of the body.

5. The apparatus according to claim 4, wherein the means for evaluating is further configured to evaluate changes in the plurality of signals that propagated through the plurality of capacitively-coupled intra body communication channels at the different instances in time in order to determine information regarding movement of the body.

6. The apparatus according to any of claim 4 or 5, wherein the means for evaluating is further configured to identify the movement of the body based upon a comparison of the information regarding the plurality of signals that propagated through the plurality of capacitively-coupled intra body communication channels at the different instances in time to a plurality of patterns associated with respective movements of the body.

7. The apparatus according to claim 6, wherein the means for evaluating is further configured to identify symmetry in the envelopes of the plurality of signals that is indicative of walking.

8. The apparatus according to any of the preceding claims, wherein the means for evaluating is further configured to identify the position of at least the portion of the body based upon a comparison of the information regarding the plurality of signals that propagated through the plurality of capacitively-coupled intra body communication channels through the body to a plurality of patterns associated with respective positions of at least the portion of the body.

9. The apparatus according to any of the preceding claims, wherein the plurality of capacitively-coupled intra body communication channels through the body comprise a plurality of capacitively-coupled intra body communication channels through the body that originate with different sources, and wherein the means for evaluating is further configured to distinguish the signals that originate with the different sources based upon a characteristic of the signals that is different for the signals transmitted by the respective sources.

10. The apparatus according to any of the preceding claims, wherein the means for evaluating comprises:
at least one processor (120); and
at least one memory (125) including computer program code, wherein the at least one memory and the computer program code are configured to, with the at least one processor, cause evaluation of the information regarding the plurality of signals in order to determine information regarding the position of at least the portion of the body.

11. The apparatus of any of the preceding claims, further comprising: means (130; 300) for transmitting the plurality of signals.

12. A method comprising:
receiving information regarding a plurality of signals that propagated between pairs of nodes (110, 115; 305, 320; 805, 810; 1105, 1100) through a plurality of capacitively-coupled intra body communication channels in a body; and
evaluating the information regarding the plurality of signals in order to determine information regarding a position of at least a portion of the body, wherein evaluating the information regarding the signal comprises identifying different positions of at least the portion of the body by evaluating envelopes (900; 1200) of the plurality of signals that propagated through the plurality of capacitively-coupled intra body communication channels that vary in a manner attributable to changes in capacitance between the nodes.

13. The method according to claim 11, wherein evaluating the information regarding the signal comprises evaluating the information regarding the plurality of signals that propagated through the plurality of capacitively-coupled intra body communication channels at different instances in time in order to determine information regarding movement of the body.

14. The method according to claim 12, further comprising: identifying symmetry in the envelopes of the plurality of signals that is indicative of walking.

15. A computer readable medium storing a computer program comprising computer program instructions configured, when working with at least one processor, to cause an apparatus at least to perform:
receiving information regarding a plurality of signals that propagated between pairs of nodes (110, 115; 305, 320; 805, 810; 1105, 1100) through a plurality of capacitively-coupled intra body communication channels in a body; and
evaluating the information regarding the plurality of signals in order to determine information regarding a position of at least a portion of the body, wherein evaluating the information regarding the signal comprises identifying different positions of at least the portion of the body by evaluating envelopes (900; 1200) of the plurality of signals that propagated through the plurality of capacitively-coupled intra body communication channels that vary in a manner attributable to changes in capacitance between the nodes.

## Patentansprüche

1. Einrichtung (100), die Folgendes umfasst:
Mittel (120; 130; 315) zum Empfangen von Informationen über eine Vielzahl von Signalen, die über eine Vielzahl von kapazitiv gekoppelten Intrakörperkommunikationskanälen in einem Körper zwischen Paaren von Knoten (110, 115; 305, 320; 805, 810; 1105, 1100) ausgebreitet wurden; und
Mittel zum Beurteilen der Informationen über die Vielzahl von Signalen, um Informationen über eine Position von mindestens einem Abschnitt des Körpers zu bestimmen, wobei die Mittel zum Beurteilen dazu ausgelegt sind, verschiedene Positionen mindestens des Abschnitts des Körpers durch Beurteilen von Hüllkurven (900; 1200) der Vielzahl von Signalen zu identifizieren, die über die Vielzahl von kapazitiv gekoppelten Intrakörperkommunikationskanälen ausgebreitet wurden, die in einer Weise variieren, die Änderungen einer Kapazität zwischen den Knoten zuzuschreiben ist.

2. Einrichtung nach Anspruch 1, wobei die Mittel zum Beurteilen dazu ausgelegt sind, eine Stärke der Vielzahl von Signalen zu beurteilen, um Informationen über die Position mindestens des Abschnitts des Körpers zu bestimmen, wobei die Stärke der Vielzahl von Signalen auf Basis von Änderungen einer Kapazität zwischen den Knoten variiert, wenn sich mindestens der Abschnitt des Körpers in verschiedenen Positionen befindet.

3. Einrichtung nach einem der Ansprüche 1 oder 2, wobei die Mittel zum Beurteilen dazu ausgelegt sind, eine Phasenverzögerung der Vielzahl von Signalen zu beurteilen, um Informationen über die Position mindestens des Abschnitts des Körpers zu bestimmen, wobei die Phasenverzögerung der Vielzahl von Signalen auf Basis von Änderungen einer Kapazität zwischen den Knoten variiert, wenn sich mindestens der Abschnitt des Körpers in den verschiedenen Positionen befindet.

4. Einrichtung nach einem der Ansprüche 1 bis 3, wobei die Mittel zum Beurteilen dazu ausgelegt sind, die Informationen über die Vielzahl von Signalen zu beurteilen, die über die kapazitiv gekoppelten Intrakörperkommunikationskanäle zu verschiedenen Zeitinstanzen ausgebreitet wurden, um Informationen über eine Bewegung des Körpers zu bestimmen.

5. Einrichtung nach Anspruch 4, wobei die Mittel zum Beurteilen ferner dazu ausgelegt sind, Änderungen an der Vielzahl von Signalen zu beurteilen, die über die Vielzahl von kapazitiv gekoppelten Intrakörperkommunikationskanälen zu den verschiedenen Zeitinstanzen ausgebreitet wurden, um Informationen über eine Bewegung des Körpers zu bestimmen.

6. Einrichtung nach einem der Ansprüche 4 oder 5, wobei die Mittel zum Beurteilen ferner dazu ausgelegt sind, die Bewegung des Körpers auf Basis eines Vergleichs der Informationen über die Vielzahl von Signalen, die über die Vielzahl von kapazitiv gekoppelten Intrakörperkommunikationskanälen zu den verschiedenen Zeitinstanzen ausgebreitet wurden, mit einer Vielzahl von Mustern, die mit jeweiligen Bewegungen des Körpers verknüpft sind, zu identifizieren.

7. Einrichtung nach Anspruch 6, wobei die Mittel zum Beurteilen ferner dazu ausgelegt sind, eine Symmetrie in den Hüllkurven der Vielzahl von Signalen zu identifizieren, die ein Gehen anzeigt.

8. Einrichtung nach einem der vorhergehenden Ansprüche, wobei die Mittel zum Beurteilen ferner dazu ausgelegt sind, die Position mindestens des Abschnitts des Körpers auf Basis eines Vergleichs der Informationen über die Vielzahl von Signalen, die über die Vielzahl von kapazitiv gekoppelten Intrakörperkommunikationskanälen durch den Körper ausgebreitet wurden, mit einer Vielzahl von Mustern, die mit jeweiligen Positionen mindestens des Abschnitts des Körpers verknüpft sind, zu identifizieren.

9. Einrichtung nach einem der vorhergehenden Ansprüche, wobei die Vielzahl von kapazitiv gekoppelten Intrakörperkommunikationskanälen durch den Körper eine Vielzahl von kapazitiv gekoppelten Intrakörperkommunikationskanälen durch den Körper umfassen, die ihren Ursprung an verschiedenen Quellen haben, und wobei die Mittel zum Beurteilen ferner dazu ausgelegt sind, die Signale, die ihren Ursprung an verschiedenen Quellen haben, auf Basis einer Charakteristik der Signale zu unterscheiden, die sich für die Signale, die von den jeweiligen Quellen übertragen werden, unterscheidet.

10. Einrichtung nach einem der vorhergehenden Ansprüche, wobei die Mittel zum Beurteilen Folgendes umfassen:
mindestens einen Prozessor (120); und
mindestens einen Speicher (125), der einen Computerprogrammcode beinhaltet, wobei der mindestens eine Speicher und der Computerprogrammcode dazu ausgelegt sind, mit dem mindestens einen Prozessor eine Beurteilung der Informationen über die Vielzahl von Signalen zu veranlassen, um Informationen über die Position mindestens des Abschnitts des Körpers zu bestimmen.

11. Einrichtung nach einem der vorhergehenden Ansprüche, die ferner Folgendes umfasst: Mittel (130; 300) zum Übertragen der Vielzahl von Signalen.

12. Verfahren, das Folgendes umfasst:
Empfangen von Informationen über eine Vielzahl von Signalen, die über eine Vielzahl von kapazitiv gekoppelten Intrakörperkommunikationskanälen in einem Körper zwischen Paaren von Knoten (110, 115; 305, 320; 805, 810; 1105, 1100) ausgebreitet wurden; und
Beurteilen der Informationen über die Vielzahl von Signalen, um Informationen über eine Position von mindestens einem Abschnitt des Körpers zu bestimmen, wobei das Beurteilen der Informationen über die Signale das Identifizieren von verschiedenen Positionen mindestens des Abschnitts des Körpers durch Beurteilen von Hüllkurven (900; 1200) der Vielzahl von Signalen umfasst, die über die Vielzahl von kapazitiv gekoppelten Intrakörperkommunikationskanälen ausgebreitet wurden, die in einer Weise variieren, die Änderungen einer Kapazität zwischen den Knoten zuzuschreiben ist.

13. Verfahren nach Anspruch 11, wobei das Beurteilen der Informationen über das Signal das Beurteilen der Informationen über die Vielzahl von Signalen umfasst, die über die Vielzahl von kapazitiv gekoppelten Intrakörperkommunikationskanälen zu verschiedenen Zeitinstanzen ausgebreitet wurden, um Informationen über eine Bewegung des Körpers zu bestimmen.

14. Verfahren nach Anspruch 12, das ferner Folgendes umfasst: Identifizieren einer Symmetrie in den Hüllkurven der Vielzahl von Signalen, die ein Gehen anzeigt.

15. Computerlesbares Medium, auf dem ein Computerprogramm gespeichert ist, das Computerprogrammanweisungen umfasst, die dazu ausgelegt sind, wenn sie mit dem mindestens einen Prozessor arbeiten, die Einrichtung veranlassen, mindestens Folgendes durchzuführen:
Empfangen von Informationen über eine Vielzahl von Signalen, die über eine Vielzahl von kapazitiv gekoppelten Intrakörperkommunikationskanälen in einem Körper zwischen Paaren von Knoten (110, 115; 305, 320; 805, 810; 1105, 1100) ausgebreitet wurden; und
Beurteilen der Informationen über die Vielzahl von Signalen, um Informationen über eine Position von mindestens einem Abschnitt des Körpers zu bestimmen, wobei das Beurteilen der Informationen über die Signale das Identifizieren von verschiedenen Positionen mindestens des Abschnitts des Körpers durch Beurteilen von Hüllkurven (900; 1200) der Vielzahl von Signalen umfasst, die über die Vielzahl von kapazitiv gekoppelten Intrakörperkommunikationskanälen ausgebreitet wurden, die in einer Weise variieren, die Änderungen einer Kapazität zwischen den Knoten zuzuschreiben ist.

## Revendications

1. Appareil (100) comprenant :
des moyens (120 ; 130 ; 315) pour recevoir des informations concernant une pluralité de signaux qui se sont propagés entre des paires de nœuds (110, 115 ; 305, 320 ; 805, 810 ; 1105, 1100) à travers une pluralité de canaux de communication intra-corps couplés de manière capacitive dans un corps ; et
des moyens pour évaluer les informations concernant la pluralité de signaux afin de déterminer des informations concernant une position d'au moins une partie du corps, dans lequel les moyens d'évaluation sont configurés pour identifier différentes positions d'au moins la partie du corps en évaluant les enveloppes (900 ; 1200) de la pluralité de signaux qui se sont propagés à travers la pluralité de canaux de communication intra-corps couplés de manière capacitive qui varient d'une manière attribuable aux changements de capacité entre les nœuds.

2. Appareil selon la revendication 1, dans lequel les moyens d'évaluation sont configurés pour évaluer une force de la pluralité de signaux afin de déterminer des informations concernant la position d'au moins la partie du corps, dans lequel la force de la pluralité de signaux varie sur la base des changements de capacité entre les nœuds lorsqu'au moins la partie du corps se trouve dans les différentes positions.

3. Appareil selon l'une des revendications 1 ou 2, dans lequel les moyens d'évaluation sont configurés pour évaluer un retard de phase de la pluralité de signaux afin de déterminer des informations concernant la position d'au moins la partie du corps, dans lequel le retard de phase de la pluralité de signaux varie sur la base des changements de capacité entre les nœuds lorsqu'au moins la partie du corps se trouve dans les différentes positions.

4. Appareil selon l'une des revendications 1 à 3, dans lequel les moyens d'évaluation sont configurés pour évaluer les informations concernant la pluralité de signaux qui se sont propagés à travers les canaux de communication intra-corps couplés de manière capacitive à différentes instances dans le temps afin de déterminer des informations concernant le mouvement du corps.

5. Appareil selon la revendication 4, dans lequel les moyens d'évaluation sont en outre configurés pour évaluer les changements dans la pluralité de signaux qui se sont propagés à travers la pluralité de canaux de communication intra-corps couplés de manière capacitive aux différentes instances dans le temps afin de déterminer des informations concernant le mouvement du corps.

6. Appareil selon l'une des revendications 4 ou 5, dans lequel les moyens d'évaluation sont en outre configurés pour identifier le mouvement du corps sur la base d'une comparaison des informations concernant la pluralité de signaux qui se sont propagés à travers la pluralité de canaux de communication intra-corps couplés de manière capacitive aux différentes instances dans le temps avec une pluralité de motifs associés à des mouvements respectifs du corps.

7. Appareil selon la revendication 6, dans lequel les moyens d'évaluation sont en outre configurés pour identifier une symétrie dans les enveloppes de la pluralité de signaux qui est indicative d'une marche.

8. Appareil selon l'une des revendications précédentes, dans lequel les moyens d'évaluation sont en outre configurés pour identifier la position d'au moins la partie du corps sur la base d'une comparaison des informations concernant la pluralité de signaux qui se sont propagés à travers la pluralité de canaux de communication intra-corps couplés de manière capacitive à travers le corps avec une pluralité de motifs associés à des positions respectives d'au moins la partie du corps.

9. Appareil selon l'une des revendications précédentes, dans lequel la pluralité de canaux de communication intra-corps couplés de manière capacitive à travers le corps comprennent une pluralité de canaux de communication intra-corps couplés de manière capacitive à travers le corps qui proviennent de sources différentes, et dans lequel les moyens d'évaluation sont en outre configurés pour distinguer les signaux qui proviennent des sources différentes sur la base d'une caractéristique des signaux qui est différente pour les signaux transmis par les sources respectives.

10. Appareil selon l'une des revendications précédentes, dans lequel les moyens d'évaluation comprennent :
au moins un processeur (120) ; et
au moins une mémoire (125) comportant un code de programme informatique, dans lequel l'au moins une mémoire et le code de programme informatique sont configurés, avec l'au moins un processeur, pour provoquer l'évaluation des informations concernant la pluralité de signaux afin de déterminer des informations concernant la position d'au moins la partie du corps.

11. Appareil selon l'une des revendications précédentes, comprenant en outre : des moyens (130 ; 300) pour transmettre la pluralité de signaux.

12. Procédé comprenant les étapes suivantes :
recevoir des informations concernant une pluralité de signaux qui se sont propagés entre des paires de nœuds (110, 115 ; 305, 320 ; 805, 810 ; 1105, 1100) à travers une pluralité de canaux de communication intra-corps couplés de manière capacitive dans un corps ; et
évaluer les informations concernant la pluralité de signaux afin de déterminer des informations concernant une position d'au moins une partie du corps, dans lequel l'évaluation des informations concernant le signal comprend l'identification de différentes positions d'au moins la partie du corps en évaluant les enveloppes (900 ; 1200) de la pluralité de signaux qui se sont propagés à travers la pluralité de canaux de communication intra-corps couplés de manière capacitive qui varient d'une manière attribuable aux changements de capacité entre les nœuds.

13. Procédé selon la revendication 11, dans lequel l'évaluation des informations concernant le signal comprend l'évaluation des informations concernant la pluralité de signaux qui se sont propagés à travers la pluralité de canaux de communication intra-corps couplés de manière capacitive à différentes instances dans le temps afin de déterminer des informations concernant le mouvement du corps.

14. Procédé selon la revendication 12, comprenant en outre l'étape suivante : identifier une symétrie dans les enveloppes de la pluralité de signaux qui est indicative d'une marche.

15. Support lisible par ordinateur stockant un programme informatique comprenant des instructions de programme informatique configurées, lorsqu'elles travaillent avec au moins un processeur, pour amener un appareil à effectuer au moins ce qui suit :
recevoir des informations concernant une pluralité de signaux qui se sont propagés entre des paires de nœuds (110, 115 ; 305, 320 ; 805, 810 ; 1105, 1100) à travers une pluralité de canaux de communication intra-corps couplés de manière capacitive dans un corps ; et
évaluer les informations concernant la pluralité de signaux afin de déterminer des informations concernant une position d'au moins une partie du corps, dans lequel l'évaluation des informations concernant le signal comprend l'identification de différentes positions d'au moins la partie du corps en évaluant les enveloppes (900 ; 1200) de la pluralité de signaux qui se sont propagés à travers la pluralité de canaux de communication intra-corps couplés de manière capacitive qui varient d'une manière attribuable aux changements de capacité entre les nœuds.
